# EUROPEAN PATENT SPECIFICATION

(11) **EP 4 105 226 B1**
(45) Date of publication and mention of the grant of the patent: **26.08.2026**
(21) Application number: 20918884.6
(22) Date of filing: 24.09.2020
(51) Int. Cl.: C07K 7/06, C12N 1/20, A61K 35/74, A61P 35/00, A61K 8/64, C12R 1/465

(54) **CYCLIC PEPTIDE COMPOUND CONTAINING PIPERAZIC ACID, METHOD OF PRODUCING SAME, AND USES OF SAME**
ZYKLISCHE PEPTIDVERBINDUNG MIT PIPERAZSÄURE, VERFAHREN ZU IHRER HERSTELLUNG UND IHRE VERWENDUNGEN
COMPOSÉ PEPTIDIQUE CYCLIQUE CONTENANT DE L'ACIDE PIPÉRAZIQUE, SON PROCÉDÉ DE PRODUCTION ET SES UTILISATIONS

(30) Priority: 14.02.2020 KR 20200018559
(43) Date of publication of application: 21.12.2022
(73) Proprietor: Molgenbio Co. Ltd., Seoul 08826 (KR)
(72) Inventor: OH, Dong-Chan, Seoul 06337 (KR); SHIN, Daniel, Seoul 06676 (KR); LEE, Sang Kook, Seoul 08826 (KR); BYUN, Woong Sub, Seoul 08792 (KR)
(74) Representative: Studio Torta S.p.A.
(86) International application number: PCT/KR2020/012966
(87) International publication number: WO 2021/162194

(56) References cited:
- WO-A1-2013/168075
- KR-A- 20190 007 083
- KR-B1- 101 522 625
- US-A1- 2009 215 789
- WOONG SUB BYUN: "Antitumor Activity of Ohmyungsamycin A through the Regulation of the Skp2-p27 Axis and MCM4 in Human Colorectal Cancer Cells", JOURNAL OF NATURAL PRODUCTS, vol. 83, no. 1, 2 January 2020 (2020-01-02), US, pages 118 - 126, XP093154951, ISSN: 0163-3864, DOI: 10.1021/acs.jnatprod.9b00918
- LUIGI DI COSTANZO ET AL: "Amino acid modifications for conformationally constraining naturally occurring and engineered peptide backbones: Insights from the Protein Data Bank", BIOPOLYMERS, JOHN WILEY, HOBOKEN, USA, vol. 109, no. 10, 25 October 2018 (2018-10-25), pages n/a, XP071165707, ISSN: 0006-3525, DOI: 10.1002/BIP.23230
- SHIN DANIEL, BYUN WOONG SUB, MOON KYUHO, KWON YUN, BAE MUNHYUNG, UM SOOHYUN, LEE SANG KOOK, OH DONG-CHAN: "Coculture of Marine Streptomyces sp. With Bacillus sp. Produces a New Piperazic Acid-Bearing Cyclic Peptide", FRONTIERS IN CHEMISTRY, vol. 6, 18 October 2018 (2018-10-18), pages 1 - 12, XP055835951, DOI: 10.3389/fchem.2018.00498
- DARDIĆ DENIS, LAURO GIANLUIGI, BIFULCO GIUSEPPE, LABOUDIE PATRICIA, SAKHAII PEYMAN, BAUER ARMIN, VILCINSKAS ANDREAS, HAMMANN PETER: "Svetamycins A–G, Unusual Piperazic Acid-Containing Peptides from Streptomyces sp.", THE JOURNAL OF ORGANIC CHEMISTRY, AMERICAN CHEMICAL SOCIETY, vol. 82, no. 12, 16 June 2017 (2017-06-16), pages 6032 - 6043, XP055835955, ISSN: 0022-3263, DOI: 10.1021/acs.joc.7b00228
- MILLER ERIC D., KAUFFMAN CHRISTOPHER A., JENSEN PAUL R., FENICAL WILLIAM: "Piperazimycins: Cytotoxic Hexadepsipeptides from a Marine-Derived Bacterium of the Genus Streptomyces", THE JOURNAL OF ORGANIC CHEMISTRY, AMERICAN CHEMICAL SOCIETY, vol. 72, no. 2, 1 January 2007 (2007-01-01), pages 323 - 330, XP055835956, ISSN: 0022-3263, DOI: 10.1021/jo061064g

## Description

### TECHNICAL FIELD

The present disclosure relates to cyclic peptide compound containing piperazic acid, method of producing the same, and anticancer use thereof.

### BACKGROUND ART

Colorectal cancer is a disease that causes ulcers and bleeding due to malignant transformation of polyps in the internal mucous membrane of the large intestine when left unattended for a long period of time. The incidence of colorectal cancer ranks second among all cancers, and the incidence rate is rapidly increasing in Korea as well, as diet becomes westernized and nutritional status improves. However, anticancer drugs generally used for treating colorectal cancer, such as irinotecan, 5-fluorouracil (5-Fu), oxaliplatin, and capecitabine, cause serious side effects such as myelosuppression, neurotoxicity, and diarrhea, and use thereof in long-term treatment may lead to resistance thereto. In recent years, cocktail therapies have been used for colorectal cancer, administering combination of anticancer drugs, but such anticancer therapy also shows severe side effects, and eventually cause resistance in long-term treatment and in recurrent cancer, and thus the use is limited.

Therefore, there is a need for a colorectal cancer treatment having a new mechanism. KR101522625B1 and Byun et al (2020, vol. 83(1)) propose cyclic peptides with antitumor properties isolated from a Streptomyces strain.

Piperazic acid which serves as a parent ring is a rare amino acid biosynthesized from its precursor, L-ornithine. Piperazic acid has been constantly discovered in various peptides derived from natural substances ever since its first discovery in 1959 (Nat. Prod. Rep. 2019, vol.36, no.12, pp.1628-1653).

Therefore, it is necessary to explore new compounds that contain a piperazic acid parent ring and show an excellent anticancer efficacy.

The present invention provides a peptide compound, a Streptomyces sp. PC5 strain, a method and a use thereof as defined in the appended claims.

Any other aspects, configurations or embodiments set forth herein not falling within the scope of the claims are for information purposes only.

As used herein, the term "peptide compound" refers to a peptide containing compound. A peptide is a compound in which two or more amino acids are linked by peptide bond(s) between a carboxyl group and an amino group. Depending on the number of amino acids, a peptide is called a dipeptide, a tripeptide, a tetrapeptide, etc., and a peptide having about 10 or less peptide bonds are called an oligopeptide, and a peptide having a plurality of peptide bonds is called a polypeptide.

The peptide compound is a compound where both ends of the compound are linked to form a ring. The peptide compound may be referred to as a cyclic peptide compound.

In particular, there is provided a peptide compound represented by any one of Formulae 2 to 6. 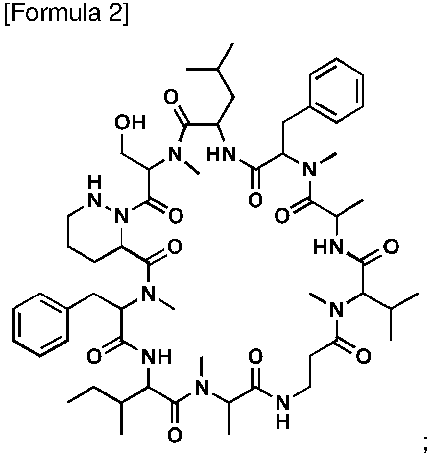 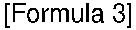 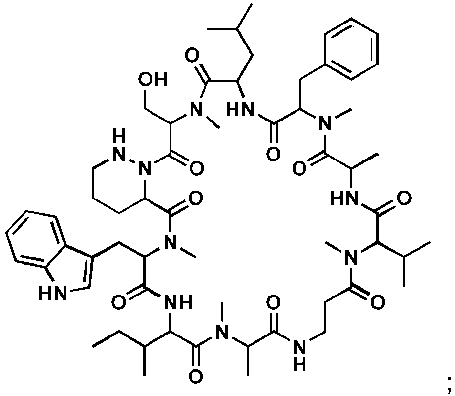 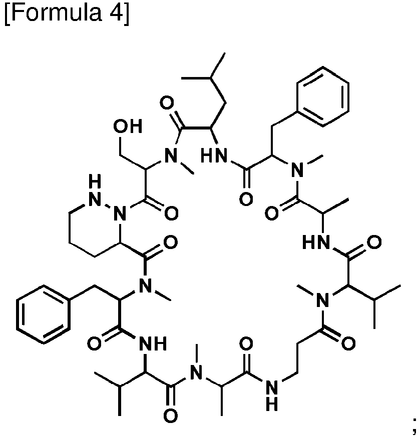 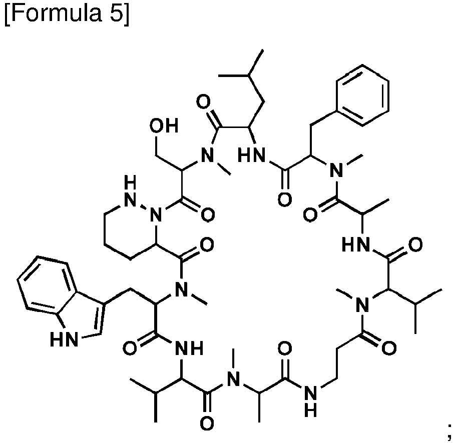 and

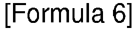

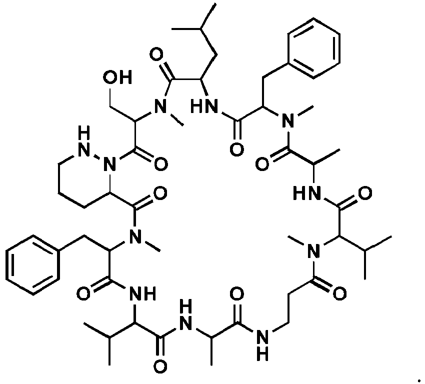

As used herein, the term "isomer" in "stereoisomer" means compounds that have the same molecular formula, but differ in the bond connections or three-dimensional arrangement of their atoms. Isomers include, for example, structural isomers and stereoisomers. The stereoisomer may be a diasteromer or an enantiomer. Enantiomers refer to two isomers that are mirror images of each other and are non-superimposable, much as one's left and right hands, and are also known as optical isomers. Enantiomers are divided into R (rectus: clockwise) and S (sinister: counterclockwise) when four or more substituents are different from each other in the chirality center carbon. Diasteromers are stereoisomers which are not mirror images of each other, and are divided into cis-trans isomers whose atoms are in different orientations in space.

The term "solvate" means a compound solvated in an organic or inorganic solvent. The solvate is, for example, a hydrate.

The term "salt" refers to inorganic and organic acid addition salts of a compound. The pharmaceutically acceptable salt may be a salt that does not cause serious irritation in the organism to which the compound is administered and does not harm biological activity or physical properties of the compound. The inorganic acid salt may be hydrochloride, bromate, phosphate, sulfate, or disulfide. The organic acid salt may be formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be calcium salt, sodium salt, magnesium salt, strontium salt, or potassium salt.

Another aspect provides a Streptomyces sp. PC5 strain deposited under accession number KCTC14117BP.

The strain is capable of producing the peptide compound, stereoisomer, solvate, or pharmaceutically acceptable salt thereof according to an aspect of the invention.

The strain includes a mutant thereof. The mutant may be, for example, a mutant generated by natural mutation or artificial mutation. The artificial mutation may be generated by a physical mutagen such as ultraviolet light, or a chemical mutagen such as a base compound.

The strain includes spores, cells, or cultures of the strain.

Another aspect provides a method of preparing the peptide compound, stereoisomer, solvate, or pharmaceutically acceptable salt thereof according to an aspect of the invention, comprising: culturing a Streptomyces sp. PC5 strain (accession number: KCTC14117BP); separating from the culture the peptide compound, stereoisomer, solvate, or pharmaceutically acceptable salt thereof according to the aspect.

The Streptomyces sp. PC5 strain, the peptide compound, stereoisomer, solvate, or pharmaceutically acceptable salt thereof are as described above.

The method includes culturing a Streptomyces sp. PC5 strain (accession number: KCTC14117BP). The culturing may be culturing the strain in a liquid medium or a solid medium. The medium may include, for example, glucose, starch syrup, dextrin, starch, molasses, animal oil, or vegetable oil as carbon sources. The medium may include, for example, bran, soybean cake, wheat, malt, cottonseed meal, fish scrap, corn steep liquor, gravy, yeast extract, malt extract, ammonium sulfate, sodium nitrate, or urea.

The culturing may be shaken or static under an aerobic condition. A cultivation temperature may be, for example, about 20 °C to about 40 °C, about 25 °C to about 37 °C, about 28 °C to about 35 °C, or about 30 °C. Cultivation duration may be, for example, about 1 day to about 4 months, about 1 day to about 2 months, about 1 day to about 6 weeks, about 1 day to about 1 month, about 1 day to about 2 weeks, or about 1 day to about 1 week.

The method includes separating the peptide compound , stereoisomer, solvate, or pharmaceutically acceptable salt thereof from the culture.

The separating may include concentration, centrifugation, filtration, or chromatography of the culture fluid. Chromatography may be, for example, column chromatography, planar chromatography, paper chromatography, or thin film chromatography according to the stationary phase. Chromatography may be, for example, gas chromatography, liquid chromatography, or affinity chromatography, depending on the physical properties of the mobile phase. Liquid chromatography may be, for example, high-performance liquid chromatography (HPLC). Chromatography may be, for example, ion exchange chromatography, size-exclusion chromatography, according to the separation method. Chromatography may be, for example, normal-phase chromatography or reverse-phase chromatography.

Another aspect provides a pharmaceutical composition for preventing or treating cancer or metastasis of cancer comprising the peptide compound, stereoisomer, solvate, or pharmaceutically acceptable salt thereof according to an aspect of the invention.

The peptide compound, stereoisomer, solvate, or pharmaceutically acceptable salt thereof are as described above.

The cancer may be solid cancer or non-solid cancer. Solid cancer refers to a cancerous growth in organs such as liver, lung, breast, and skin. Non-solid cancer is cancer in the blood and is also called hematological cancer. The cancer may be, for example, intrahepatic cholangiocarcinoma, liver cancer, thyroid cancer, colon cancer, testicular cancer, myelodysplastic syndrome, glioblastoma, oral cancer, mycosis fungoides, acute myeloid leukemia, chronic myeloid leukemia, acute lymphocytic leukemia, chronic lymphocytic leukemia, basal cell carcinoma, ovarian epithelial cancer, ovarian germ cell tumor, male breast cancer, brain tumor, pituitary adenoma, multiple myeloma, gallbladder cancer, biliary tract cancer, colorectal cancer, retinoblastoma, choroidal melanoma, ampulla of Vater cancer, bladder cancer, peritoneal cancer, parathyroid cancer, adrenal cancer, non-small cell lung cancer, tongue cancer, astrocytoma, small cell lung cancer, pediatric brain tumor, pediatric lymphoma, pediatric leukemia, small intestine cancer, meningioma, esophageal cancer, glioma, neuroblastoma, pelvic ureter cancer, renal cancer, malignant soft tissue tumor, malignant bone tumor, malignant lymphoma, malignant mesothelioma, malignant melanoma, eye tumor, vulvar cancer, urethral cancer, cancer of unknown primary site, gastric lymphoma, gastric cancer, gastric carcinoid tumor, gastrointestinal stromal tumor, Wilms tumor, breast cancer, sarcoma, penile cancer, pharyngeal cancer, gestational trophoblastic disease, cervical cancer, endometrial cancer, uterine sarcoma, prostate cancer, metastatic brain tumor, rectal cancer, rectal carcinoid tumor, vaginal cancer, spinal cord tumor, acoustic neuroma, pancreatic cancer, salivary gland cancer, tonsil cancer, squamous cell carcinoma, lung adenocarcinoma, lung cancer, lung squamous cell carcinoma, skin cancer, anal cancer, laryngeal cancer, or a combination thereof. The cancer may be, for example, lung cancer, colorectal cancer, breast cancer, liver cancer, stomach cancer, or leukemia.

Metastasis refers to spread of cancerous cells to a different tissue away from the primary site. Metastasis may be a progressed form of epithelial-mesenchymal transition (EMT).

The peptide compound, stereoisomer, solvate, or pharmaceutically acceptable salt thereof may be used for cell cycle regulation of cancer cells, apoptosis induction of cancer cells, metastasis inhibition, or a combination thereof. Cell cycle regulation of cancer cells may be G2/M phase arrest or sub G1 phase accumulation.

The cancer may have resistance to an anticancer agent. The term "anticancer agent" or "anticancer drug" as used in "resistance to an anticancer agent" refers to chemicals used for treatment of malignant tumors including reduction, inhibition, and removal of malignant tumor. The anticancer agent may be, for example, alkylating agents, antimetabolites, natural materials, hormones, and hormone antagonists, targeted therapeutic agents, or combinations thereof. The anticancer agent is, for example, fluorouracil (5-fu), irinotecan, etoposide, gemcitabine, and paclitaxel. The term "drug-resistance" refers to a symptom of showing no signs of mitigation, reduction, alleviation or treatment of cancers in response to an anticancer drug therapy due to an extremely low susceptibility thereto Cancer cells may have an innate resistance to a certain anticancer agent, or may acquire resistance as the cancer cells change in the course of a long-term treatment with a specific anticancer agent and no longer show susceptibility for the anticancer agent to which it had been susceptible at first.

The term "prevention" refers to all actions that inhibit development or progression of a disease or delay the onset of a disease by administrating a composition. The term "treatment" refers to all actions that alleviate symptoms of a disease or change symptoms in a beneficial way by administrating a composition.

The pharmaceutical composition may further comprise a known active ingredient having an anticancer activity. The known active ingredient having an anticancer activity may be an anticancer drug. The anticancer agent may be 5-fluorouracil (5-Fu), irinotecan, etoposide, oxaliplatin, leucovorin, capecitabine, or combination thereof. The peptide compound, stereoisomer, solvate, or pharmaceutically acceptable salt thereof, and the anti-cancer agent may be in a single composition or separate compositions for simultaneous or sequential administrations.

The pharmaceutical composition may further include a carrier, an excipient or a diluent. Carriers, excipients and diluents may include, for example, lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, or mineral oil.

The pharmaceutical composition may be formulated as oral formulations such as powders, granules, tablets, capsules, suspensions, emulsions, syrups, and aerosols, or as external preparations, suppositories, or sterilized solutions for injection. In the formulation, the pharmaceutical composition may be prepared by using a diluent or excipient such as a filler, a thickening agent, a binder, a wetting agent, a disintegrant, or a surfactant.

In the pharmaceutical composition, solid formulations for oral administration may be tablets, pills, powders, granules, or capsules. The solid formulation may further include an excipient. The excipient may be, for example, starch, calcium carbonate, sucrose, lactose, or gelatin. In addition, the solid formulation may further comprise a lubricant such as magnesium stearate, or talc. In the pharmaceutical composition, liquid formulations for oral administration may be a suspension, oral liquids, an emulsion, or a syrup. The liquid formulation may include water or liquid paraffin. The liquid formulation may include an excipient, for example, a wetting agent, a sweetener, a fragrance, or a preservative. In the pharmaceutical composition, formulations for parenteral administration may be sterile aqueous solutions, non-aqueous solvents, suspensions, emulsions, lyophilized preparations, or suppositories. The non-aqueous solvents or the suspensions may include, vegetable oil or ester. The vegetable oil may be, for example, propylene glycol, polyethylene glycol, or olive oil. The ester may be ethyl oleate. As a base for suppositories, Witepsol, Macrogol, Tween 61, cacao butter, laurin butter, glycerogelatin may be used.

The pharmaceutical composition may include the compound, stereoisomer, solvate, or pharmaceutically acceptable salt thereof, in an effective amount. The term "effective amount" refers to the amount of an agent that is sufficient to lead to a desired effect of prevention or treatment when administered to a subject in need thereof. The effective amount may be selected by those skilled in the art depending on the cell or the subject. The effective amount of the pharmaceutical composition may vary depending upon the subject's condition and body weight, severity of the disease, formulation of the drug, route and duration of administration, etc., and may be appropriately selected by those skilled in the art. However, the compound, stereoisomer, solvate, or pharmaceutically acceptable salt thereof may be administered in an amount of, for example, about 0.0001 mg/kg to about 100 mg/kg, or about 0.001 mg/kg to about 100 mg / kg, 1 to 24 times a day, or 1 to 7 times in 2 days or a week, or 1 to 24 times in 1 month to 12 months. In the pharmaceutical composition, the compound, stereoisomer, solvate, or pharmaceutically acceptable salt thereof may be included in an amount of about 0.0001 wt% to about 10 wt% or about 0.001 wt% to about 1 wt% with respect to the total weight of the composition.

The administration may be oral, or parenteral administration. The administration may be via for example, oral, intradermal, subcutaneous, rectal, intravenous, intra-arterial, intraperitoneal, intramuscular, intrasternal, local, intranasal, intratracheal, or intradermal route. The composition may be administered systemically or locally, and the composition may be administered alone or in combination with other pharmaceutically active compounds.

A further aspect provides functional food comprising the compound, stereoisomer, solvate, or pharmaceutically acceptable salt thereof according to an aspect of the invention, for prevention or improvement of bacterial infection caused by Mycobacterium sp. or any related symptoms.

The compound, stereoisomer, solvate, and pharmaceutically acceptable salt thereof, bacterial infection caused by Mycobacterium sp. or any related symptoms, and prevention are as described above.

The term "improvement" includes all actions that alleviate symptoms of a disease or lead to beneficial effects.

The functional food may be used as functional food by formulating the peptide compound, stereoisomer, solvate, or salt thereof into capsules, powders, suspensions, or etc., or by adding to various food products. The food products include, for example, meat, sausages, bread, chocolate, candy, snack, confectionery, pizza, ramen, other noodles, chewing gums, dairy products including ice cream, various soups, beverages, teas, drinks, alcoholic beverages, vitamin supplements, functional food and health food.

A further aspect provides a peptide compound, stereoisomer, solvate, or pharmaceutically acceptable salt thereof as defined in claim 1 for use in preventing or treating cancer or metastasis of cancer.

The peptide compound, cancer, metastasis of cancer, prevention, and treatment are as described above.

The subject may be a mammal, for example, a human, a mouse, a rat, a cow, a horse, a pig, a dog, a monkey, a sheep, a goat or a cat. The subject may have cancer or a symptom related to metastasis of cancer, or be at risk of suffering from cancer.

The use may further include administering an anticancer agent to the subject. The anticancer agent may be administered to the subject simultaneously, separately, or sequentially with the compound, stereoisomer, solvate, or pharmaceutically acceptable salt thereof.

The administration may be oral, or parenteral administration. The administration may be via, for example, oral, intradermal, subcutaneous, rectal, intravenous, intra-arterial, intraperitoneal, intramuscular, intrasternal, local, intranasal, intratracheal, or intradermal route. The peptide compound may be administered systemically or locally, and may be administered alone or in combination with other pharmaceutically active compounds.

A preferred dose of the peptide compound may vary depending upon the subject's condition and weight, severity of the disease, formulation of the drug, route and duration of administration, etc., and may be appropriately selected by those skilled in the art. The dose for an adult may be in the range of, for example, about 0.001 mg/kg to about 100 mg/kg, about 0.01 mg/kg to about 10 mg/kg, or about 0.1 mg/kg to about 1 mg/kg. The administration may be carried out once a day, several times a day, once a week, once in 2 weeks, once in 3 weeks, or once in 4 weeks to once a year.

### ADVANTAGEOUS EFFECTS OF DISCLOSURE

The novel piperazic acid containing peptide compound, stereoisomer, solvate, or pharmaceutically acceptable salt thereof have activities of anticancer, anti-metastasis, and growth inhibition of resistant tumors, and thus may be used to prevent or treat various cancers or metastases of cancer.

### BRIEF DESCRIPTION OF DRAWINGS

FIG. 1 is a photograph of a solid medium on which a Streptomyces sp. PC5 strain was cultivated;
FIG. 2A is graphs showing cell cycles of a colorectal cancer cell line HCT116 depending on the concentration (µM) of lenziamide A, with the cell cycle measured by flow cytometry, and FIG. 2B is a graph showing the cell cycle distribution (%) of HCT116 depending on the concentration of lenziamide A (µM);
FIG. 3A is flow cytometry graphs showing results of Annexin V/propidium iodide dual-stain analysis, and FIG. 3B is a graph showing cell stages depending of the concentration of lenziamide A;
FIG. 4A is a graph showing tumor volumes (mm³) with the administration of lenziamide A, FIG. 4B is a graph showing body weights (g) of xenograft animal models, and FIG. 4C is a graph showing final tumor weight (g) depending on the amount of lenziamide A administered;
FIG. 5A is a graph showing tumor volumes (mm³) in resistant cancer cell line injected xenograft animal models depending on the administration of lenziamide A, 5-FU, or combination thereof, and FIG. 5B is a graph showing the weights (g) of the resistant cancer cell line injected xenograft animal models;
FIG. 6A is an image of immunoblotting analysis showing the expression of metastasis related biomarkers in a metastatic colorectal cancer cell line depending on the administration of lenziamide A, FIG. 6B is a graph showing tumor volumes (mm³) in metastatic colorectal cancer cell injected xenograft animal models with the administration of lenziamide A, and FIG. 6C is a graph showing body weights of metastatic colorectal cancer cell injected xenograft animal models.

### MODE OF DISCLOSURE

Hereinafter, the disclosure will be described in more detail by using the following examples. However, these examples are only intended to illustrate the present disclosure, and the scope of the present disclosure is as defined in the appended claims.

### Example 1. Isolation of lenziamide and identification of structure thereof

### 1-1. Isolation of lenziamide-producing strain

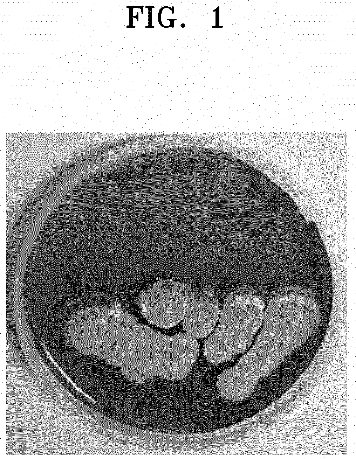

A Lenziamide-producing Streptomyces sp. PC5 strain was isolated from an integument of *Onthophagus lenzii.* PC5 strain was isolated from Actinomycete Isolation solid medium (2 g of sodium caseinate, 0.1 g of asparagine, 4 g of sodium propionate, 0.5 g of dipotassium phosphate, 0.1 g of magnesium sulfate, 0.001 g of ferrous sulfate and 15 g of agar powder per 1 L of sterile water) (FIG.1). Based on 16S rDNA sequence analysis results, the strain was identified to be a Streptomyces species. The strain was named Streptomyces sp. PC5 strain, and the strain was deposited to Korea Research Institute of Bioscience and Biotechnology on January 29th, 2020 (accession number KCTC1411BP).

### 1-2. Culture of Streptomyces sp. PC5 strain

A Streptomyces sp. PC5 strain was plated on sterilized yeast extract-malt extract (YEME) solid medium (4 g of yeast extract, 10 g of malt extract, 4 g of glucose, and 18 g of agar per 1 L of distilled water) and cultured for about 4 weeks at about 30 °C.

The spores of the cultured PC5 strain were inoculated to a 50 mL volume of YEME liquid medium (4 g of yeast extract, 10 g of malt extract, and 4 g of glucose per 1 L of distilled water), and cultured for about 8 days at 30°C with shaking at 200 rpm. 5 mL of the culture fluid was inoculated to a 200 mL volume of modified YEME liquid medium (4 g of yeast extract, 10 g of malt extract, 4 g of glucose, and 1 g of humic acid per 1 L of distilled water) and cultured for about 5 days at 30 °C with shaking at 170 rpm.

### 1-3. Isolation and purification of lenziamide

### Culture fluid of PC5 strain was obtained from Example 1-2.

1 L of PC5 strain culture fluid obtained at the end of the culturing and about 1.5 L volume of ethyl acetate (EtOAc, Daejung Hwageum Co., Ltd.) were poured into a separatory funnel which was mounted on a stand, and the funnel was closed with a stopper and shook for 3 minutes up and down and right and left to conduct a primary extraction. Afterwards, the separatory funnel was again mounted on a stand for complete separation of a water layer and an EtOAc layer, and then the valve of the separator funnel was opened to remove the water layer. New EtOAc was added to the water layer, and iterative extractions were carried out in the same manner. The EtOAc layers were stored separately in a clean flask, and anhydrous sodium sulfate (Daejung Hwageum Co., Ltd.) was added thereto to remove the remaining water from the culture fluid, and the solution was filtered through several layers of clean gauzes to remove impurities. The dehydrated EtOAc layer was transferred to a 3 L round bottom flask and dried under a reduced pressure in a vacuum dry oven. A total of 150 L of culture fluid was used to obtain 5 g of crude extract by extraction.

A reverse-phase fractionation was first carried out by using an open column to purify lenziamide from the PC5 strain extract. After stabilizing the open column filled with C₁₈ resin with 20 % (v/v) methanol (MeOH)/ 80 % (v/v) H₂O, the extract adsorbed on Celite 545 was filled thereon. The extract was fractionated by each of 20 % (v/v), 40 % (v/v), 60 % (v/v), 80 % (v/v), 100 % (v/v) methanol solvent. Fractions of 80 % (v/v) and 100 % (v/v) methanol solvent where lenziamide A was distributed were transferred to a round bottom flask and dried under a reduced pressure.

In order to obtain pure lenziamide A from dried 80 % (v/v) and 100 % (v/v) methanol fractions, high-performance liquid chromatography (HPLC) was performed, followed by further purification in three steps below.
(1) Fractionation was conducted with use of a reverse-phase column (C₁₈ YMC-Pack ODS-A S-5 µm 250 x 10.0 mm) and elution with concentration gradient from 50 % (v/v) to 80% (v/v) acetonitrile (ACN) to which 0.1 % (v/v) of formic acid is added / aqueous solution (flow rate: 2 mL/min, detection: UV 210 nm) for about 50 minutes. Lenziamide A, B, C, D, and E were respectively identified in fractions obtained at about 31-minute, about 26-minute, about 28-minute, and about 23- minute.

(A-2) The 31-minute fraction dried under a reduced pressure was further fractionated by using a reverse-phase column (C₁₈ YMC-Pack ODS-A S-5 µm 250 x 10.0 mm) under the isocratic condition of 76 % (v/v) methanol (MeOH) to which 0.1 % (v/v) of formic acid is added / aqueous solution (flow rate: 2 mL/min, detection: UV 230 nm). Lenziamide A was identified in a fraction at about 28-minute.

(A-3) The 28-minute fraction dried under a reduced pressure was further fractionated under the same condition as in (A-2) to obtain pure lenziamide A. By conducting iterative experiments, about 100 mg of lenziamide A was obtained from about 5 g of crude extract.

(B-2) The 26-minute fraction dried under a reduced pressure was further fractionated for 40 minutes by using a cyano column (CN YMC-Pack S-5 µm 250 x 4.6 mm) with a concentration gradient of 40 % (v/v) to 70 % (v/v) methanol (MeOH) to which 0.1 % (v/v) of formic acid is added / aqueous solution (flow rate: 0.8 mL/min, detection: UV 230 nm). Pure lenziamide B was obtained in a fraction at about 25-minute.

(C-2) The 28-minute fraction dried under a reduced pressure was further fractionated for about 40 minutes by using a cyano column (CN YMC-Pack S-5 µm 250 x 4.6 mm) with a concentration gradient of 40 % (v/v) to 70 % (v/v) methanol (MeOH) to which 0.1 % (v/v) of formic acid is added / aqueous solution (flow rate: 0.8 mL/min, detection: UV 230 nm). Pure lenziamide C was obtained in a fraction at about 27-minute.

(D-2) The about 23-minute fraction dried under a reduced pressure was further fractionated for about 40 minutes by using a cyano column (CN YMC-Pack S-5 µm 250 x 4.6 mm)with a concentration gradient of 40 % (v/v) to 70 % (v/v) methanol (MeOH) to which 0.1 % (v/v) of formic acid is added / aqueous solution (flow rate: 0.8 mL/min, detection: UV 230 nm). Pure lenziamide D was obtained from a fraction at about 23-minute, and lenziamide E was identified mixed with impurities from a fraction at about 21-minute.

(E-3) The about 21-minute fraction obtained in the course of obtaining pure lenziamide D and dried under a reduced pressure was further fractionated by using a reverse-phase column (C₁₈ (2) Luna 5 µm 250 x 4.6 mm) with the isocratic condition of 73 % (v/v) methanol (MeOH) to which 0.1 % (v/v) of formic acid is added / aqueous solution (flow rate: 2 mL/min, detection: UV 230 nm). Lenziamide E was obtained in a fraction at about 21-minute.

### 1-4. Identification of chemical structure of lenziamide

The structure of lenziamide A was identified based on 1D and 2D nuclear magnetic resonance(NMR) spectrum. For the nuclear magnetic resonance spectrum (¹H NMR, ¹³C NMR), Bruker's 500 MHz NMR was used, and acetone-*d*₆ was used as a solvent.

The positioning of the structure of lenziamide A by the nuclear magnetic resonance spectrum is shown in Table 1.

### [Lenziamide A]

(1) Molecular formula: C₅₇H₈₇N₁₁O₁₁
(2) Molecular weight: 1101
(3) Color: Transparent
(4) ¹H-NMR (Acetone-*d*₆, 850 MHz): Refer to Table 1
(4) ¹³C-NMR (Acetone-d₆, 212.5 MHz): Refer to Table 1

**[Table 1]**

| Amino acid | Position | δ_{C} | Type | δ_{H} | mult. (*J* in Hz) |
|---|---|---|---|---|---|
| Ile | 1 | 174.5 | C | | |
| | 2 | 53.9 | CH | 4.78 | dd (11.0, 8.5) |
| | 2-NH | | | 7.73 | d (8.5) |
| | 3 | 35.5 | CH | 2.36 | m |
| | 4 | 25.5 | CH₂ | 1.43 | m |
| | | | | 1.18 | m |
| | 5 | 9.6 | CH₃ | 0.86 | t (7.5) |
| | 6 | 14.8 | CH₃ | 1.01 | d (6.5) |
| N-Me-Phe | 7 | 169.6 | C | | |
| | 8 | 63.3 | CH | 4.50 | dd (11.5, 1.5) |
| | 9a | 34.7 | CH₂ | 2.85 | m |
| | 9b | | | 2.52 | d (14.0) |
| | 10 | 139.0 | C | | |
| | 11 | 130.1 | CH | 6.41 | d (5.5) |
| | 12 | 129.4 | CH | 7.10 | m |
| | 13 | 127.3 | CH | 7.11 | m |
| | 14 | 129.4 | CH | 7.10 | m |
| | 15 | 130.1 | CH | 6.41 | d (5.5) |
| | 16 | 28.5 | CH₃ | 2.77 | s |
| Pip | 17 | 172.3 | C | | |
| | 18 | 46.4 | CH | 4.35 | dd (5.5, 3.5) |
| | 19a | 23.3 | CH₂ | 0.49 | m |
| | 19b | | | 0.06 | m |
| | 20a | 19.4 | CH₂ | 1.59 | m |
| | 20b | | | 0.90 | m |
| | 21a | 47.0 | CH₂ | 2.96 | m |
| | 21b | | | 2.67 | m |
| | 21-NH | | | 4.77 | dd (13.0, 2.0) |
| N-Me-Ser | 22 | 171.1 | C | | |
| | 23 | 69.9 | CH | 4.41 | m |
| | 24a | 62.1 | CH₂ | 4.21 | ddd (12.0, 12.0, 7.0) |
| | 24b | | | 3.77 | |
| | | | | | m |
| | 24-OH | | | 4.91 | dd (11.5, 2.0) |
| | 25 | 39.1 | CH₃ | 2.96 | s |
| Leu | 26 | 174.6 | C | | |
| | 27 | 48.4 | CH | 4.96 | ddd (10.0, 10.0, 4.0) |
| | 27-NH | | | 8.79 | d (9.5) |
| | 28a | 41.4 | CH₂ | 1.67 | ddd (14.5, 10.5, 4.0) |
| | 28b | | | 1.38 | |
| | | | | | m |
| | 29 | 24.8 | CH | 1.57 | m |
| | 30 | 24.2 | CH₃ | 0.90 | d (7.0) |
| | 31 | 22.5 | CH₃ | 0.91 | d (7.0) |
| N-Me-Phe | 32 | 171.3 | C | | |
| | 33 | 59.0 | CH | 6.00 | dd (12.5, 3.0) |
| | 34a | 37.2 | CH₂ | 3.58 | dd (14.5, 3.5) |
| | 34b | | | 3.04 | dd (14.5, 12.5) |
| | 35 | 139.9 | C | | |
| | 36 | 129.6 | CH | 7.29 | d (7.5) |
| | 37 | 129.4 | CH | 7.33 | dd (7.5, 7.5) |
| | 38 | 127.6 | CH | 7.23 | d (7.5) |
| | 39 | 129.4 | CH | 7.33 | dd (7.5, 7.5) |
| | 40 | 129.6 | CH | 7.29 | d (7.5) |
| | 41 | 32.4 | CH₃ | 3.33 | s |
| Ala | 42 | 176.3 | C | | |
| | 43 | 45.0 | CH | 4.84 | qd (9.0, 7.0) |
| | 43-NH | | | 6.74 | d (8.5) |
| | 44 | 18.0 | CH₃ | 0.69 | d (7.0) |
| N-Me-Val | 45 | 170.1 | C | | |
| | 46 | 62.4 | CH | 4.58 | d (10.0) |
| | 47 | 25.1 | CH | 2.22 | m |
| | 48 | 19.9 | CH₃ | 0.91 | d (6.5) |
| | 49 | 18.6 | CH₃ | 0.78 | d (6.5) |
| | 50 | 30.0 | CH₃ | 2.74 | s |
| -Ala | 51 | 173.6 | C | | |
| | 52a | 33.6 | CH₂ | 2.64 | ddd (16.5, 5.5,2.0) |
| | 52b | | | 2.42 | |
| | | | | | ddd (16.5, 10.5, 2.5) |
| | 53a53b | 35.1 | CH₂ | 3.57 | m |
| | | | | 3.37 | m |
| | 53-NH | | | 7.23 | m |
| N-Me-Ala | 54 | 172.2 | C | | |
| | 55 | 52.7 | CH | 5.62 | q (7.0) |
| | 56 | 16.5 | CH₃ | 1.39 | d (7.5) |
| | 57 | 31.9 | CH₃ | 3.42 | s |

The chemical structure of lenziamide A analyzed from the nuclear magnetic resonance spectrum data and the stereo-structure analysis result is shown below.

### [Lenziamide A]

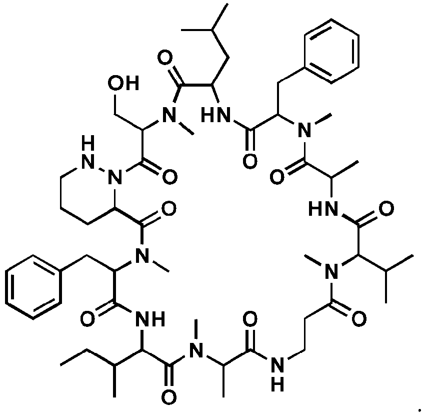

On the other hand, the chemical structures of lenziamides B to E are shown below.

### [Lenziamide B]

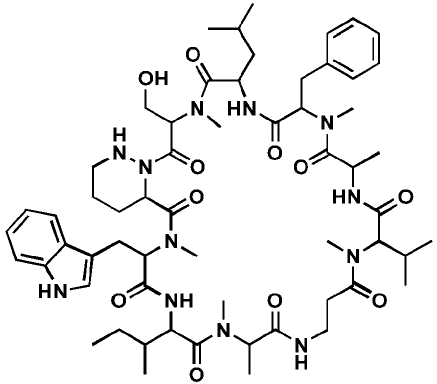

### [Lenziamide C]

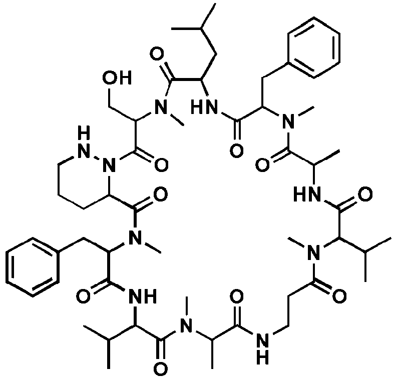

### [Lenziamide D]

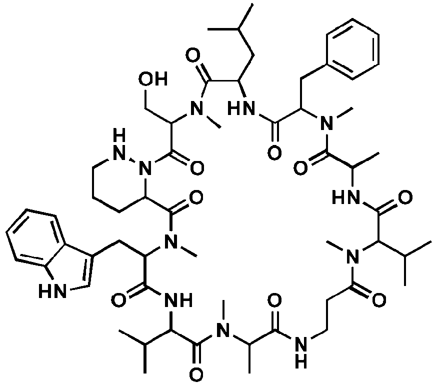

### [Lenziamide E]

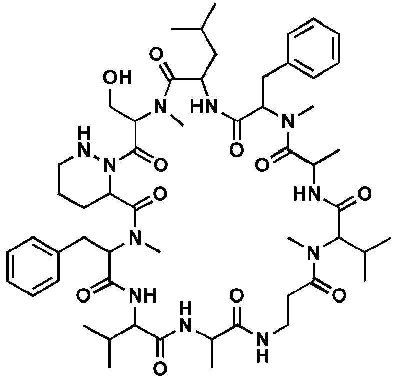

### Example 2. Identification of anticancer activity of lenziamide

### 2-1. Evaluation of growth inhibitory activity in cancer cells

6 types of cancer cell lines of Lung adenocarcinoma cell A549, colorectal cancer cell HCT116, breast cancer cell MDA-MB-231, liver adenocarcinoma cell SK-HEP-1, stomach adenocarcinoma cell SNU638, and chronic myeloid leukemia cell K562, and a normal cell line of pulmonary fibroblast MRC-5 were purchased from Korean Cell Line Bank (Seoul, Korea).

With Lenziamide compound prepared as in Example 1 added, the cell lines were cultured for about 72 hours. Thereafter, the resulting cell cultures were stained with sulforhodamine (SRB) that only stains live cells and washed to remove non-binding dye, and the stained cells were suspended in 10 mM of Tris (pH 10.0). The absorbance was measured at 515 nm and the cell proliferation was measured. Using TableCurve 2D v5.01 software (Systant Software Inc., Richmond, CA, USA), half maximal inhibitory concentration (IC₅₀) value was calculated by nonlinear regression analysis method. All reagents were purchased from Sigma-Aldrich. The calculated IC₅₀ values (µM) are shown in Table 2. As a positive control, etoposide was used.

**[Table 2]**

| IC₅₀(µm) | A549 | HCT116 | MDA-MB-231 | SK-HEP-1 | SNU638 | K562 | MRC-5 |
|---|---|---|---|---|---|---|---|
| Lenziamide A | 0.80 | 0.37 | 0.62 | 0.39 | 0.44 | 0.77 | >20 |
| Lenziamide B | 0.43 | 0.20 | 0.23 | 0.22 | 0.25 | 0.36 | >20 |
| Lenziamide C | 0.76 | 0.52 | 0.96 | 0.53 | 0.43 | 0.65 | >20 |
| Lenziamide E | 1.78 | 1.01 | 1.59 | 0.96 | 1.32 | 1.21 | >20 |
| Etoposide | 0.59 | 1.86 | 3.36 | 0.45 | 0.48 | 0.76 | 11.57 |

As shown in Table 2, lenziamide compounds were found to effectively inhibit growth of cancer cells.

### 2-2. Cell cycle control efficacy of lenziamide A in colorectal cancer cells.

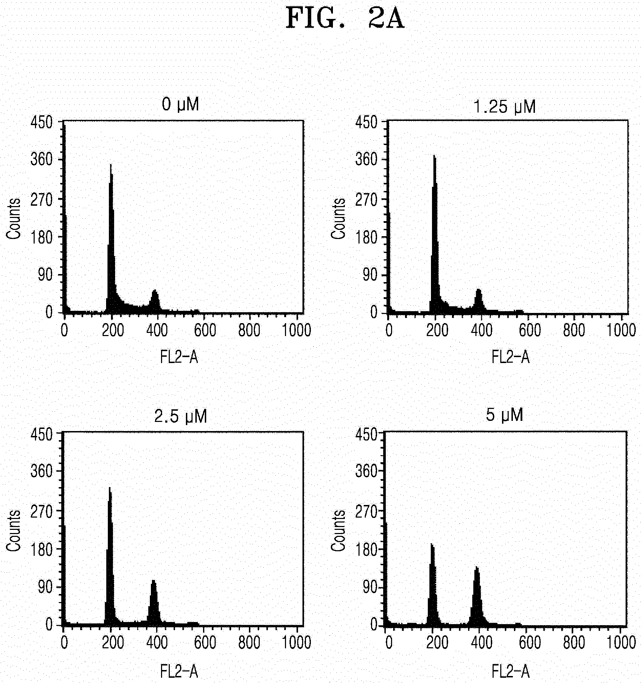

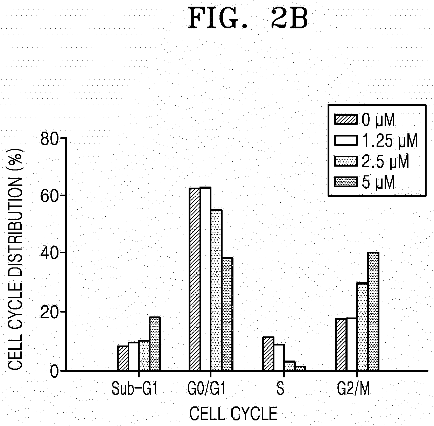

In order to identify the action mechanism of lenziamide Ashowing an excellent cell proliferation inhibiting efficacy, cells of colorectal cancer cell line HCT116 were treated with lenziamide A at a concentration of 0 µM, 1.25 µM, 2.5 µM, or 5 µM. The cell cycle control effect of lenziamide A was confirmed by using flow cytometry method, and the results are shown in FIGS. 2A and 2B.

As shown in FIGS. 2A and 2B, G2/M cell cycle arrest was increased until 24 hours after treatment of lenziamide A, followed by sub G1 cell accumulation, which is cell death in the presence of a high concentration of lenziamide. That is, it was found that lenziamide A shows inhibitory efficacy of cancer cell proliferation through cell death by inducing G2/M cell cycle arrest.

### 2-3. Apoptosis induction efficacy of lenziamide A in colorectal cancer cells

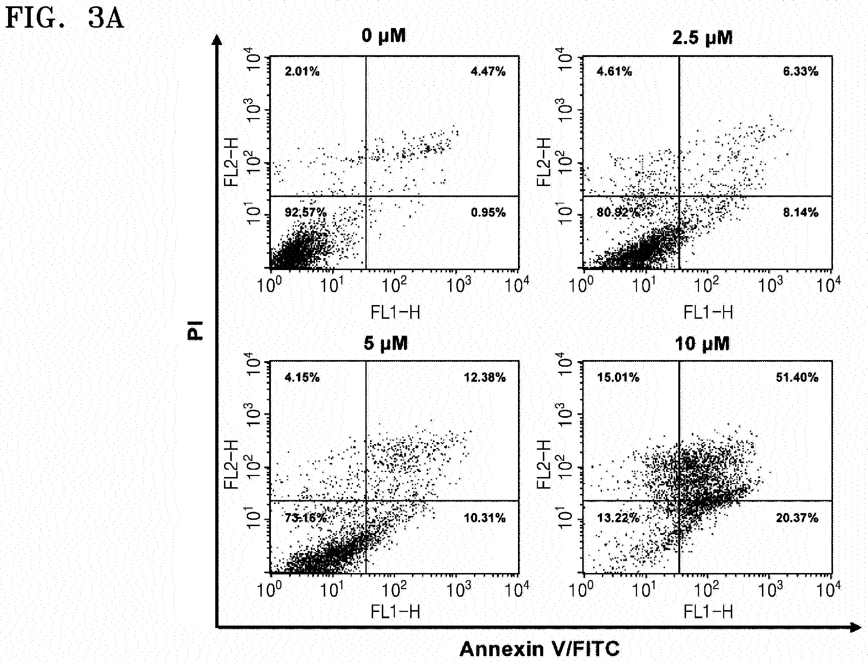

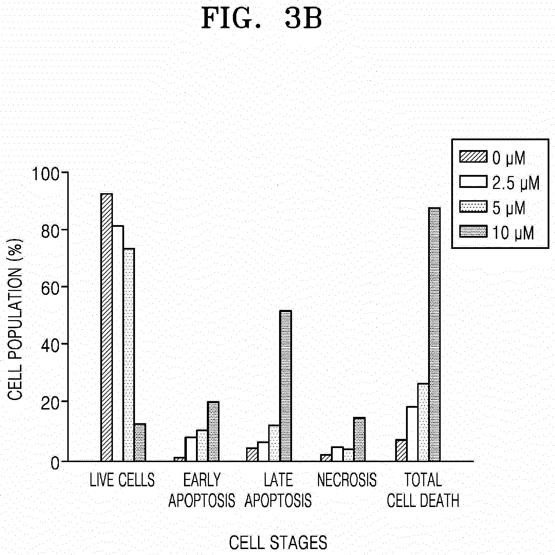

In order to confirm that sub G1 cell accumulation due to G2/M arrest effect of lenziamide A leads to cell apoptosis, HCT116 cells were treated with different concentrations of lenziamide A. Thereafter, cell death induction efficacy of lenziamide A was evaluated by using Annexin V/propidium iodide dual-stain analysis method which can identify cell deaths. The results of Annexin V/propidium iodide dual-stain analysis were shown in FIG. 3A, and stages of cells according to the concentration of lenziamide A were shown in FIG. 3B. The ratios (%) of live cells, early apoptosis, late apoptosis, necrosis, and total cell death were calculated.

As shown in FIGS. 3A and 3B, apoptosis increased dependent on the concentration of lenziamide A. Particularly, when 5 µM of lenziamide A was treated, a population of about 20.37 % of early apoptosis and 51.40% of late apoptosis cells was shown. Thus, lenziamide A was confirmed to inhibit growth of cancer cells by inducing cell apoptosis.

### 2-4. In vivo growth inhibitory efficacy of lenziamide A in colorectal cancer cells

In order to evaluate antitumor efficacy of lenziamide A in an animal model, xenograft animal models were prepared by grafting colorectal cancer cells.

Cells of HCT116, a human-derived colorectal cancer cell line, were diluted to a concentration of 5 x 10⁶ units / mL in RPMI (Roswell Park Memorial Institute) medium, and 0.2 mL of the diluted cells was injected subcutaneously to right flank of BALB/c mice (5-week old, male). When the size of the tumor reached about 100 mm³, groups were randomly divided based on the size of the tumor.

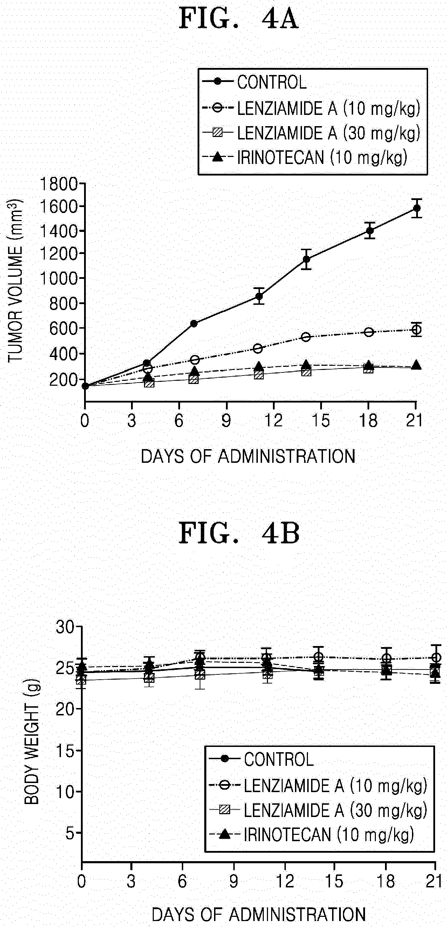

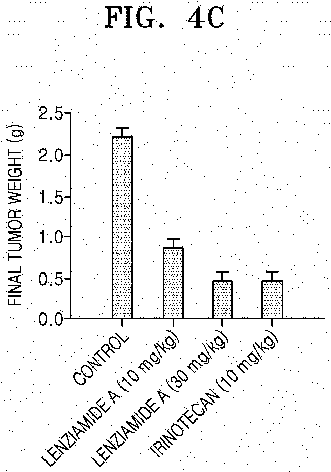

Lenziamide A was administered by intravenous injection at a dose of 10 mg/body weight kg or 30 mg/body weight kg to the prepared xenograft animal models. A mixture mixed at a ratio of dimethyl sulfoxide: Cremophor: saline = 0.5: 0.5: 9 was used as a negative control, and irinotecan was used as a positive control. Volume of the tumor (mm³), body weight of the xenograft animal model (g), and final weight of the tumor (g) were measured from the first day of administration, and the results are shown in FIGS. 4A to 4C.

As shown in FIGS. 4A to 4C, a concentration-dependent tumor growth inhibitory effect was found at the two concentrations (10, 30 mg/kg) of lenziamide A. On the other hand, toxicity caused by lenziamide A was evaluated with body weight (g) during the administration period, and no significant weight decrease was observed.

### 2-5. Growth inhibitory efficacy of lenziamide A in a 5-fluorouracil resistant-colorectal cancer cell line

In order to evaluate efficacy of lenziamide A in resistant cell lines, HCT116-5-FU, a colorectal cancer cell line resistant to 5-fluorouracil (5-Fu), which is used as the primary therapeutic agent for colorectal cancer, was prepared.

In order to prepare HCT116-5-FU resistant cell line, 5-fluorouracil was treated to HCT116 cells 3 times a week, and only surviving cells were subcultured with treatment of increasing concentrations of 5-fluorouracil for 3 months. HCT116-5-FU resistant cell line was established by finally collecting and culturing only the surviving cells.

5-Fu, irinotecan, etoposide, and lenziamide A were added to HCT116 cell line without resistance and HCT116-5-FU resistant cell line, and half maximal inhibitory concentration (IC₅₀) values were calculated as described in Example 2-1. IC₅₀ values (µM) according to the compound, and fold change of IC₅₀ value of HCT116-5-FU with respect to IC₅₀ value of HCT116 were calculated, and the results are shown in Table 3.

**[Table 3]**

| IC₅₀(µM) | HCT116 | HCT116-5-FU | Fold change (HCT116-5-FU/ HCT116) |
|---|---|---|---|
| 5-fluorouracil | 4.22 | 36.70 | 8.70 |
| Irinotecan | 1.52 | 5.65 | 3.72 |
| Etoposide | 0.76 | 1.92 | 2.53 |
| Lenziamide A | 0.36 | 0.42 | 1.16 |

As shown in Table 3, HCT116-5-FU resistant cell line was found resistant to other drugs used for treating colorectal cancer as well as 5-FU, but lenziamide A effectively inhibited growth of HCT116-5-FU resistant cell line. Thus, lenziamide A was confirmed to have an effective growth inhibition effect on resistant cancer cells.

### 2-6. In vivo growth inhibitory efficacy of lenziamide A in resistant tumor cells and efficacy of lenziamide A in combination with 5-FU.

In order to verify efficacy of lenziamide A at an animal experiment level, xenograft animal models were developed using a resistant cancer cell line.

HCT116-5-FU resistant cell line prepared as described in Example 2-5 was diluted to a concentration of 5 x 10⁶ units/mL in RPMI medium, and 0.2 mL of the diluted cell line was injected subcutaneously to right flanks of BALB/c mice (5-week old, male). When the size of the tumor reached about 100 mm³, groups were randomly divided based on the size of the tumor.

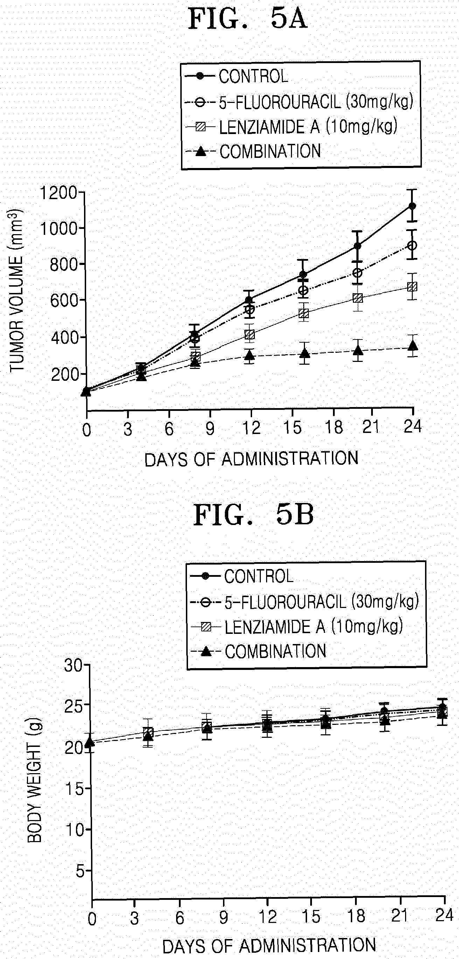

Lenziamide A (10 mg/ body weight kg), 5-FU (30 mg/ body weight kg), or a combination thereof (10 mg/body weight kg of lenziamide A + 30 mg/body weight kg of 5-FU) was administered by intravenous injection to resistant cancer cell line injected xenograft animal models. A mixture mixed at a ratio of dimethyl sulfoxide: Cremophor: saline = 0.5 : 0.5: 9 was used as a negative control. Volumes of tumor (mm³) and body weights of the xenograft animal models (g) were measured from the first day of administration, and the results are shown in FIGS. 5A and 5B.

As shown in FIGS. 5A and 5B, when lenziamide A is administered at a dose of 10 mg/kg, lenziamide A effectively inhibited growth of resistant tumor without significant weight loss. Further, in the case of a combined administration of lenziamide A and 5-FU, a synergistic effect was observed compared to administration of 5-FU alone. Thus, lenziamide A was confirmed to have an in vivo growth inhibitory effect for resistant cells, and show a synergistic effect when administered in combination with other anti-cancer agents such as 5-FU.

### 2-7. Anti-metastasis efficacy of lenziamide A

### (1) Identification of in vitro anti-metastasis efficacy

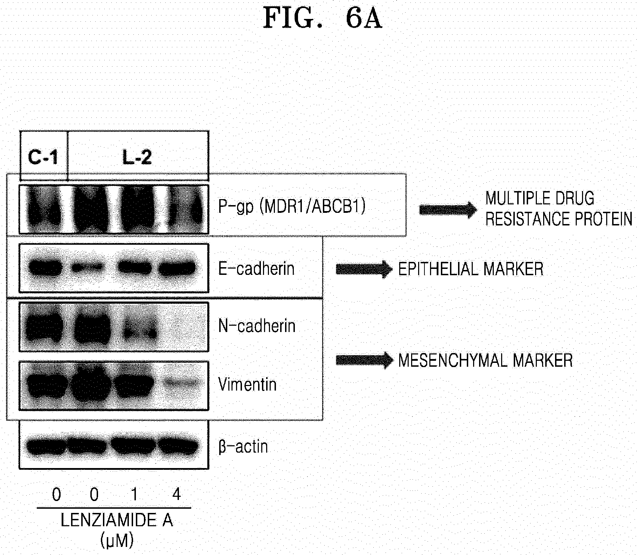

In general, metastatic cancer is known to have faster growth rate that that of non-metastatic cancer, thereby making it very difficult to treat it. A colorectal cancer cell line (C-1) was injected into appendix of laboratory mice and left for 6 weeks to artificially induce growth and metastasis of tumor, and colorectal cancer cells metastasized to the liver (L-2) were isolated and cultured. After administering 0 µM to 4 µM of lenziamide A to colorectal cancer cell line (C-1) and metastatic colorectal cancer cell line (L-2) obtained above, expression of biomarkers related to metastasis of cancer was identified by reverse transcription polymerase chain reaction (RT-PCR), and the results are shown in FIG. 6A.

As shown in FIG. 6A, metastatic colorectal cancer cells (L-2) had a reduced expression of E-cadherin, an epithelial biomarker, compared to a colorectal cancer cell line (C-1), but had an increased expression of N-Cadherin and vimentin, which are biomarkers related to metastasis of cancer. When lenziamide A was treated to colorectal cancer cells metastasized to liver (L-2), expression of E-cadherin increased, while expression of N-Cadherin and vimentin decreased. Thus, lenziamide A was confirmed to have anti-metastasis efficacy in vitro.

### (2) Identification of in vivo anti-metastasis efficacy

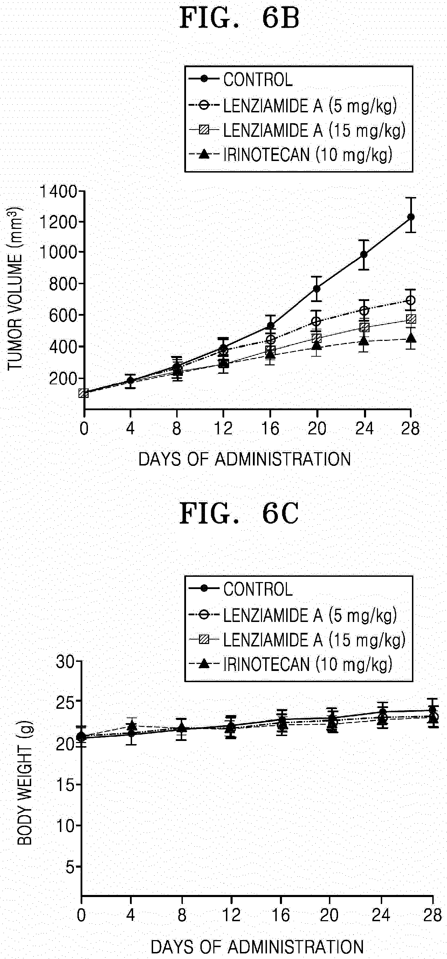

As described in Example 2-7 (1), a metastatic colorectal cancer cell line (L-2) was re-injected into appendix of laboratory mice to form tumor, and lenziamide A was administered at a dose of 5 mg/body weight kg or 15 mg/body weight kg. A mixture mixed at a ratio of dimethyl sulfoxide: Cremophor: saline = 0.5: 0.5: 9 was used as a negative control. 10 mg/body weight kg of irinotecan was administered as a positive control. Volumes of tumor (mm³) and body weights of the xenograft animal model (g) were measured from the first day of administration, and the results are shown in FIGS. 6B and 6C.

As shown in FIGS. 6B and 6C, in groups to which 5 mg/kg or 15 mg/kg of lenziamide A was administered, metastasis of the tumor to other organs was effectively inhibited, and growth of tumor in the appendix was also inhibited. Thus, lenziamide A was confirmed to have excellent anti-metastasis efficacy in vivo.

### <Accession number>

Name of Depositary Authority: Korea Research Institute of Bioscience and Biotechnology
Accession number: KCTC14117BP
Date of Receipt: 20200129

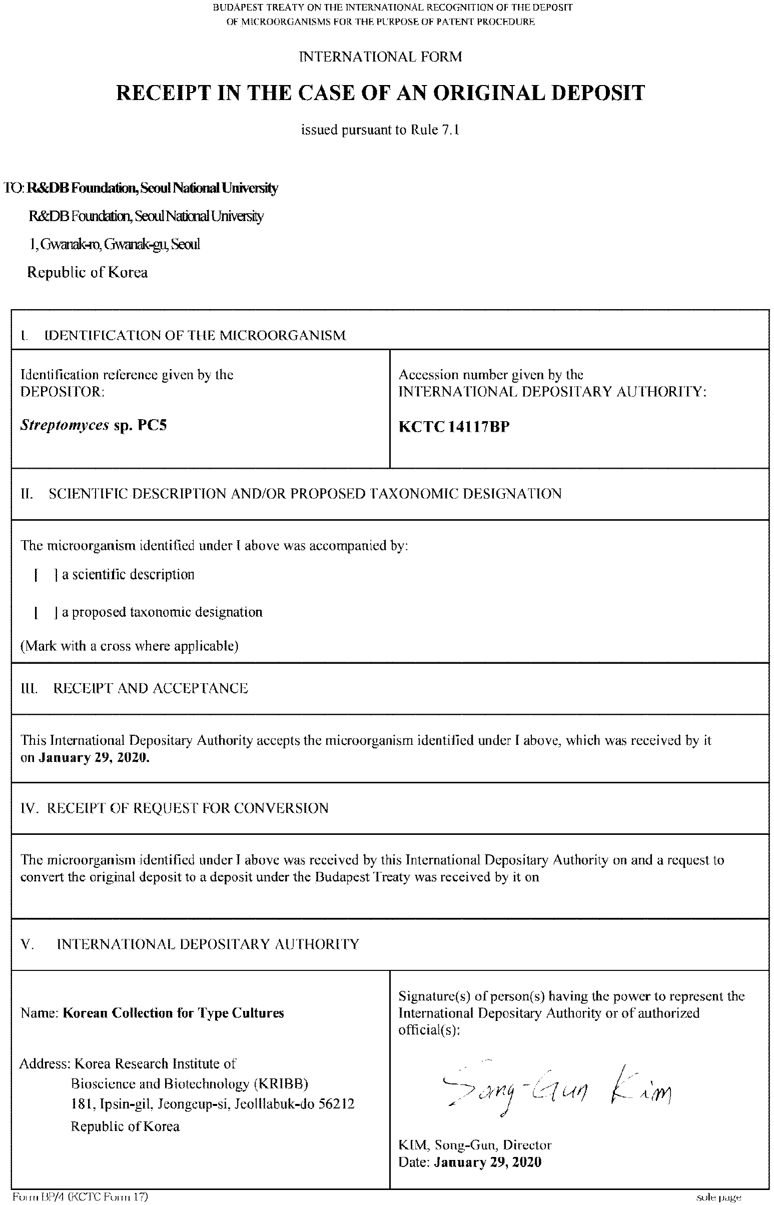
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: R&DB Foundation, Seoul National University
R&DB Foundation, Seoul National University
1, Gwanak-ro, Gwanak-gu, Seoul
Republic of Korea

I. IDENTIFICATION OF THE MICROORGANISM
Identification reference given by the
DEPOSITOR:

Streptomyces sp. PC5 | Accession number given by the
INTERNATIONAL DEPOSITARY AUTHORITY:

KCTC 14117BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on January 29, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: January 29, 2020
Form BP/4 (KCTC Form 17) sole page

## Claims

1. A peptide compound, stereoisomer, solvate, or pharmaceutically acceptable salt thereof, wherein the peptide compound is represented by any one of Formulae 2 to 6: 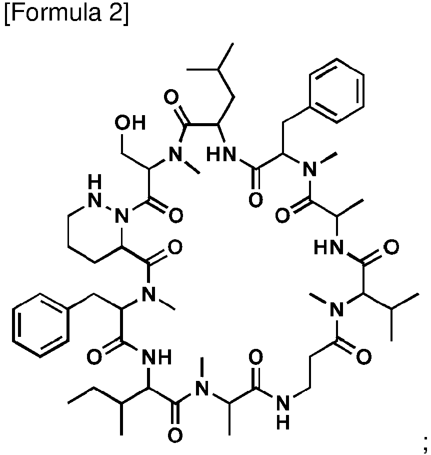 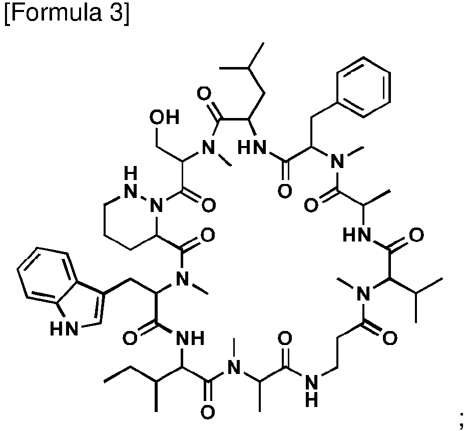 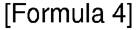 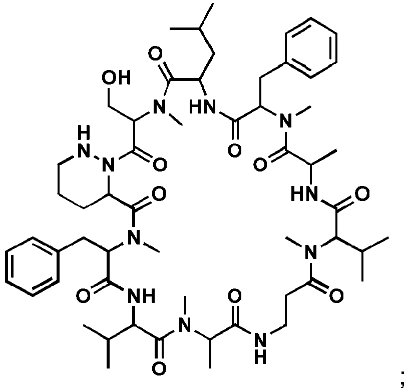 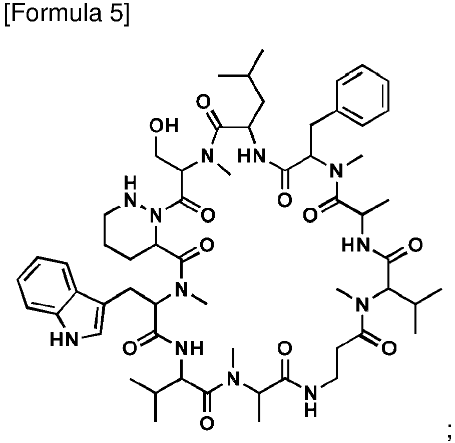 and

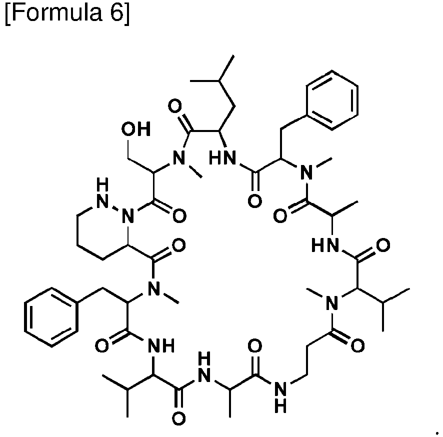

2. Streptomyces sp. PC5 strain capable of producing the peptide compound of claim 1, stereoisomer, solvate, or pharmaceutically acceptable salt thereof and deposited in the KCTC Korean Collection for Type Culture) under accession number KCTC14117BP.

3. A method of preparing the peptide compound of claim 1, stereoisomer, solvate, or pharmaceutically acceptable salt thereof, the method comprising:
culturing a Streptomyces sp. PC5 strain deposited under accession number KCTC14117BP; and
separating the peptide compound, stereoisomer, solvate, or pharmaceutically acceptable salt thereof, from the culture.

4. The peptide compound of claim 1, stereoisomer, solvate, or pharmaceutically acceptable salt thereof for use in preventing or treating cancer or metastasis of cancer.

## Patentansprüche

1. Peptidverbindung, Stereoisomer, Solvat oder pharmazeutisch verträgliches Salz davon, wobei die Peptidverbindung durch eine der Formeln 2 bis 6 dargestellt ist: 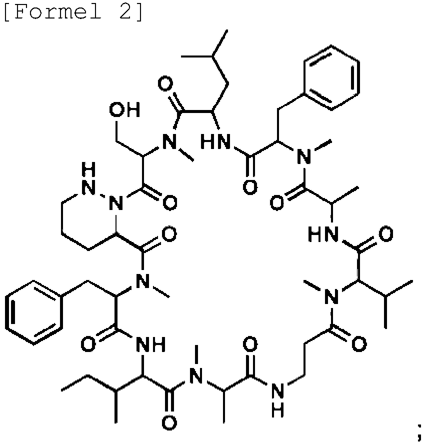 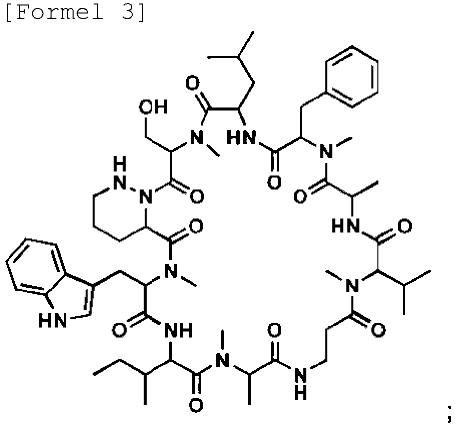 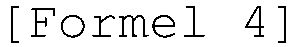 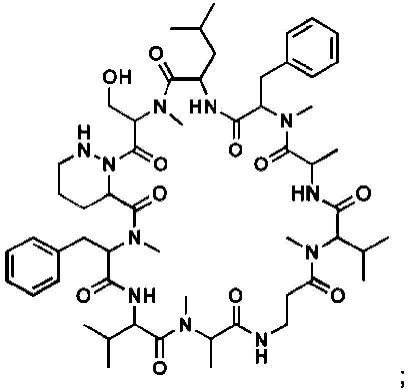 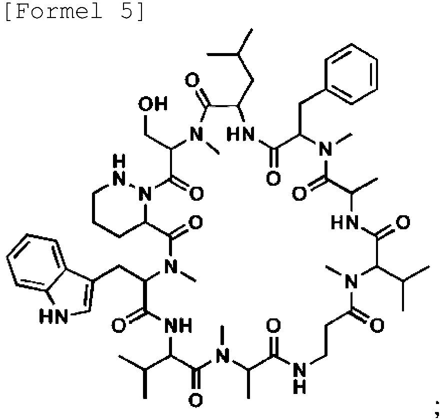 und

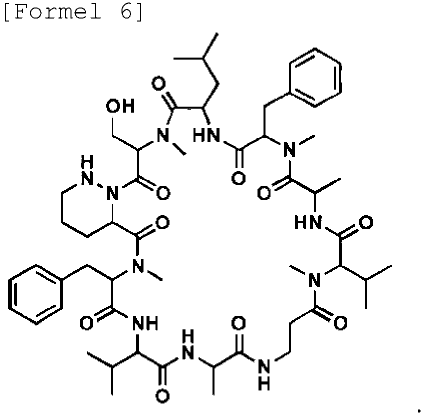

2. Streptomyces-sp.- PC5-Stamm, der in der Lage ist, die Peptidverbindung nach Anspruch 1, das Stereoisomer, das Solvat oder das pharmazeutisch verträgliche Salz davon herzustellen und der in der KCTC (Korean Collection for Type Culture) unter der Zugangsnummer KCTC14117BP hinterlegt ist.

3. Verfahren zur Herstellung der Peptidverbindung nach Anspruch 1, des Stereoisomers, Solvats oder pharmazeutisch verträglichen Salzes davon, wobei das Verfahren Folgendes umfasst:
Kultivieren eines Streptomyces-sp.- PC5-Stamms, der unter der Zugangsnummer KCTC14117BP hinterlegt ist, und
Trennen der Peptidverbindung, des Stereoisomers, des Solvats oder des pharmazeutisch verträglichen Salzes davon aus der Kultur.

4. Peptidverbindung nach Anspruch 1, Stereoisomer, Solvat oder pharmazeutisch verträgliches Salz davon zur Verwendung bei der Vorbeugung oder Behandlung von Krebs oder Krebsmetastasen.

## Revendications

1. Composé peptidique, stéréoisomère, solvate ou sel pharmaceutiquement acceptable de celui-ci, dans lequel le composé peptidique est représenté par l'une quelconque des formules 2 à 6 : 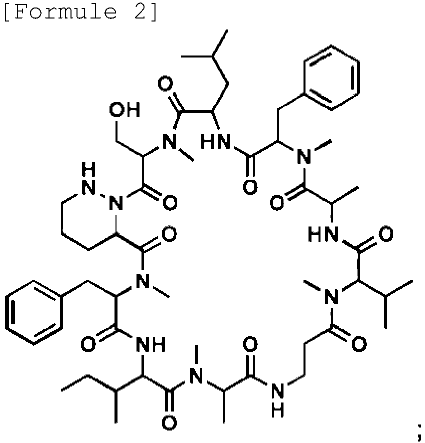 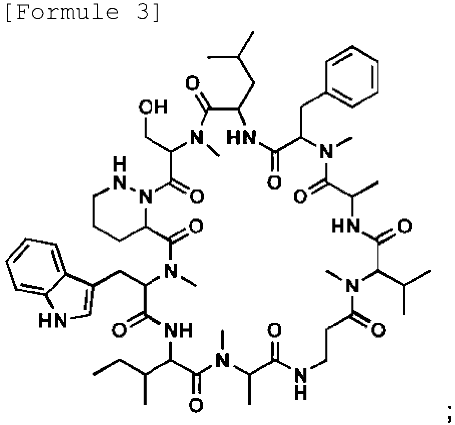 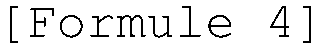 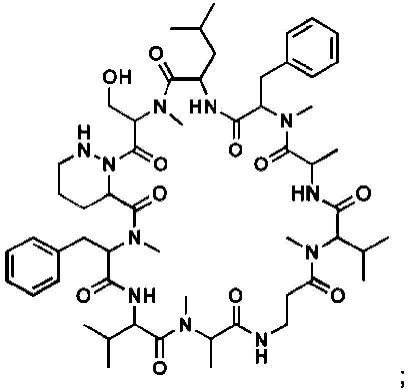 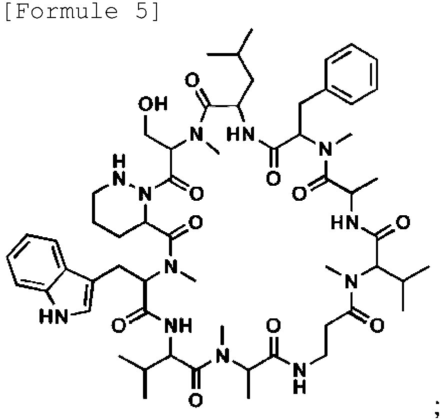 et

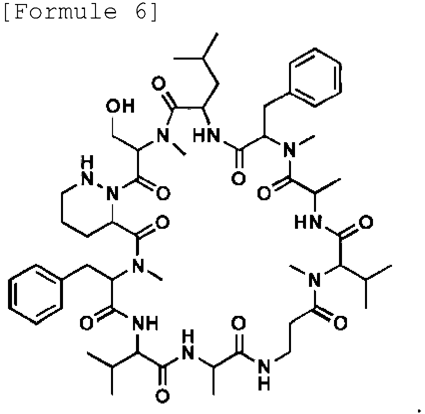

2. Souche de Streptomyces sp. PC5 pouvant produire le composé peptidique selon la revendication 1, son stéréoisomère, son solvate ou son sel pharmaceutiquement acceptable, et déposé auprès de la KCTC (Korean Collection for Type Culture) sous le numéro d'accès KCTC14117BP.

3. Procédé de préparation du composé peptidique selon la revendication 1, de son stéréoisomère, de son solvate ou son sel pharmaceutiquement acceptable, le procédé comprenant :
la culture d'une souche de Streptomyces sp. PC5 déposée sous le numéro d'accès KCTC14117BP ; et
la séparation du composé peptidique, son stéréoisomère, son solvate ou son sel pharmaceutiquement acceptable de la culture.

4. Composé peptidique selon la revendication 1, son stéréoisomère, son solvate ou son sel pharmaceutiquement acceptable, destiné à être utilisé dans la prévention ou le traitement du cancer ou des métastases cancéreuses.
